(19) Europäisches Patentamt
European Patent Office
Office européen des brevets

(11) Publication number: 0 355 234 A1

# (12) EUROPEAN PATENT APPLICATION

(21) Application number: 88402163.5

(51) Int. Cl.[4]: A61K 31/44

(22) Date of filing: 25.08.88

(43) Date of publication of application: 28.02.90 Bulletin 90/09

(84) Designated Contracting States: AT BE CH DE ES FR GB GR IT LI LU NL SE

(71) Applicant: MERRELL DOW PHARMACEUTICALS INC.
2110 East Galbraith Road
Cincinnati Ohio 45215-6300(US)

(72) Inventor: Di Francesco, Giuseppe F.
8 Rond Point de L'Esplanade
F-67000 Strasbourg(FR)
Inventor: Petty, Margaret A.
49, rue de L'Yser
F-67000 Strasbourg(FR)

(74) Representative: Gillard, Marie-Louise et al
Cabinet Beau de Loménie 55, Rue d'Amsterdam
F-75008 Paris(FR)

## (54) Pyrazolopyridine derivatives as antiatherosclerotic and antihypercholesterolemic agents.

(57) This invention relates to the use of certain pyrazolopyridine derivatives in the manufacture of a medicament for treating a patient for atherosclerosis or hypercholesterolemia.

EP 0 355 234 A1

# PYRAZOLOPYRIDINE DERIVATIVES AS ANTIATHEROSCLEROTIC AND ANTIHYPERCHOLESTEROLEMIC AGENTS

Atherosclerosis as manifested in its major clinical complication, ischaemic heart disease, continues to be a major cause of death in industrialized countries. It is now well accepted that atherosclerosis can begin with local injury to the arterial endothelium followed by proliferation of arterial smooth muscle cells from the medial layer to the intimal layer along with deposition of lipid in the lesion and calcification of the atherosclerotic plaque. As the atherosclerotic plaque develops it progressively occludes more and more of the affected blood vessel and can eventually lead to ischaemia or infarction. Therefore, it is desirable to provide methods of preventing or inhibiting the development of atherosclerotic plaques in patients in need thereof.

Furthermore, there is now a large body of evidence demonstrating that hypercholesterolemia is an important risk factor associated with heart disease. For example, in December 1984, a National Institute of Health Consensus Development Conference Panel concluded that lowering definitely elevated blood cholesterol levels (specifically blood levels of low-density lipoprotein cholesterol) will reduce the risk of heart attacks due to coronary heart disease. Accordingly, it is desirable to provide a method for reducing plasma cholesterol in patients with hypercholesterolemia.

It has been demonstrated that certain agents interfering with calcium influx into cells - for example, lanthenum and chelators such as ethylenediamine tetraacetic acid (EDTA), phosphonates, and thiophenes -can inhibit the development of atherosclerotic plaque in rabbits or primates maintained on a hypercholesterolemic diet. Similar findings have been reported for nifedipine, verapamil, and nicardipine. However, calcium channel blockers which have been shown to have antiatherosclerotic effects have not been shown to have antihypercholesterolemic effects.

The present invention relates to the use of certain pyrazolopyridine derivatives in the treatment of patients for atherosclerosis and/or hypercholesterolemia. These pyrazolopyridine derivatives are known calcium antagonists and antihypertensive agents and are disclosed in European Patent Application 83112273.4 (Publication No. 0-114-273-A1) and in European Patent Application No. 83810442.0 (Publication No. 0-107-619-A).

More specifically, the present invention provides a method of treating a patient for atherosclerosis or hypercholesterolemia comprising administration of an effective antiatherosclerotic or antihypercholesterolemic amount of a pyrazolopyridine derivative, or a physiologically acceptable salt thereof. The present invention also provides a use of these pyrazolopyridine derivatives in the manufacture of a medicament for treating a patient for atherosclerosis or hypercholesterolemia.

The pyrazolopyridine derivatives which are useful in the present invention are known compounds described in European Patent Application No. 83112273.4 (Publication No. 0-114-273-A1) and European Application No. 83810442.0 (Publication No. 0-107-619-A1) both of which publications are incorporated herein by reference in their entirety. These pyrazolopyridine derivatives can be made by procedures which are well known and appreciated by those skilled in the art, such as those procedures described in the above-identified publications incorporated herein.

The pyrazolopyridine derivatives which are useful according to the present invention are those described by formula (1)

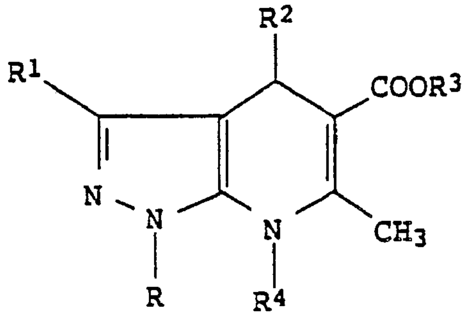

(1)

wherein

R is hydrogen, $(C_1\text{-}C_6)$alkyl, phenyl, or phenyl which is optionally substituted with from 1 to 3 substituents selected from $(C_1\text{-}C_4)$alkyl, halo$(C_1\text{-}C_4)$alkyl, $(C_1\text{-}C_4)$alkoxy, chloro, fluoro, bromo, nitro, and

$(C_1-C_6)$alkoxy-carbonyl, or phenyl$(C_1-C_4)$alkyl, wherein the phenyl group is optionally substituted as above;

$R^1$ is hydrogen, $(C_1-C_6)$alkyl, $(C_3-C_7)$cycloalkyl, phenyl, or phenyl which is optionally substituted by from 1, 2 or 3 substituents selected from $(C_1-C_4)$alkyl, halo$(C_1-C_4)$alkyl, $(C_1-C_4)$alkoxy, chloro, fluoro, bromo, nitro, and $(C_1-C_6)$alkoxy-carbonyl, or phenyl$(C_1-C_4)$alkyl, wherein the phenyl group is optionally substituted as above;

$R^2$ is a phenyl group substituted by one or two substituents selected from nitro, $(C_1-C_4)$alkoxy, $(C_1-C_4)$-alkyl, chloro or trifluoromethyl;

$R^3$ is $(C_1-C_6)$alkyl or $(C_1-C_4)$alkoxy$(C_1-C_6)$alkyl;

$R^4$ is hydrogen, methyl or benzyl; or the physiologically acceptable salts thereof.

The various terms used herein have the following meanings:

the term "patient" refers to warm-blooded animals or mammals, including humans, horses, bovine cattle, pigs, sheep, mice, rats, rabbits, cats, dogs and the like, in need of treatment for atherosclerosis or hypercholesterolemia;

the term "$(C_1-C_6)$alkyl" refers to hydrocarbyl radicals of one to six carbon atoms of straight or branched-chain configuration and includes, for example, methyl, ethyl, propyl, isopropyl, n-butyl, isobutyl, pentyl, hexyl and the like;

the term "$(C_3-C_7)$cycloalkyl" refers to hydrocarbyl radicals of three to seven carbon atoms of cyclic configuration and includes, for example, cyclopropyl, cyclobutyl, cyclopentyl, cyclohexyl, cycloheptyl and the like;

the term "$(C_1-C_6)$alkoxy" refers to alkoxy analogs of $(C_1-C_6)$alkyl radicals as defined above and includes, for example, methoxy, ethoxy, propoxy, butoxy, pentoxy, hexoxy and the like;

the terms "$(C_1-C_4)$alkyl" and "$(C_1-C_4)$alkoxy" refer to groups of one to four carbon atoms which are included in the above definitions of $(C_1-C_6)$alkyl and $(C_1-C_6)$alkoxy, respectively;

the term "halo" refers to halogen atoms selected from chloro, bromo and fluoro while the term "halo$(C_1-C_4)$alkyl" refers to halogenalkyl groups of one to four carbon atoms wherein some or all of the hydrogen atoms are replaced with halogen atoms (representative examples of halo$(C_1-C_4)$alkyl groups are:

trifluoromethyl, chlorodifluoromethyl, bromochlorofluoromethyl, trichloromethyl, 1,1-dichloroethyl, 1,2-dichloroethyl, 1-chloro-2,2,2-trifluoroethyl and the like).

Physiologically acceptable salts are those pharmaceutically acceptable salts wherein the whole toxicity of the compound is not increased compared with the non-salt. Acid addition salts are obtained by treating compounds of the formula (1) with pharmaceutically acceptable acids. Representative examples of acids suitable for the formation of physiologically acceptable salts are: hydrohalide, sulfuric, phosphoric, and nitric acids; aliphatic, alicyclic, aromatic or heterocyclic carboxylic or sulfonic acids, such as formic, acetic, propionic, succinic, glycolic, lactic, malic, tartaric, citric, ascorbic, $\alpha$-ketoglutaric, glutammic, aspartic, maleic, hydroxymaleic and pyruvic acid; phenylacetic acid, benzoic, paraaminobenzoic, anthranilic, para-hydroxybenzoic, salicylic, para-aminosalicylic or embonic acid, methanesulfonic, ethanesulfonic, hydroxyethanesulfonic, ethylenesulfonic acid; halobenzenesulfonic acid or sulfanilic acid. The preferred physiologically acceptable salt is the monohydrochloride.

Atherosclerosis is a disease state characterized by the development and growth of atherosclerotic lesions or plaque. The identification of those patients who are at risk of developing atherosclerosis and the diagnosis of atherosclerosis in a patient suffering therefrom are well within the ability and knowledge of one skilled in the art. Any person skilled in the art can therefore readily determine which individuals are patients in need of treatment for atherosclerosis.

An effective antiatherosclerotic amount of a pyrazolopyridine derivative is an amount which is effective in inhibiting development or growth of an atherosclerotic plaque. As such, successful treatment of a patient for atherosclerosis is understood to include effectively slowing, interrupting, arresting, or stopping atherosclerotic plaque development or growth and does not necessarily indicate an total elimination of the atherosclerosis. It is further understood and appreciated by those skilled in the art that successful treatment for atherosclerosis includes prophylaxis in preventing atherosclerotic plaque formation.

Hypercholesterolemia is a disease state characterized by levels of plasma cholesterol which are elevated over that considered normal by those of ordinary skill in the art. One skilled in the art can readily determine the normal or desireable range for plasma cholesterol in patients. The identification of those patients who are at risk of developing hypercholesterolemia and the diagnosis of hypercholesterolemia in a patient suffering therefrom are well within the ability and knowledge of one skilled in the art. Any person skilled in the art can therefore readily determine which individuals are patients in need of treatment for hypercholesterolemia.

An effective antihypercholesterolemic amount of a pyrazolopyridine derivative is an amount which is effective in reducing plasma cholesterol. As such, successful treatment of a patient with hypercholesterolemia is understood to include reducing plasma cholesterol to levels which are considered more normal or more desireable by one skilled in the art.

An effective antiatherosclerotic or antihypercholesterolemic dose can be readily determined by the use of conventional techniques and by observing results obtained under analogous circumstances. In determining the effective dose, a number of factors are considered including, but not limited to: the species of patient,; its size, age, and general health; the specific disease involved; the degree of or involvement or the severity of the disease; the response of the individual patient; the particular compound administered; the mode of administration; the bioavailability characteristics of the preparation administered; the dose regimen selected; and the use of concomitant medication.

An effective antiatherosclerotic or antihypercholesterolemic amount of a pyrazolopyridine derivative will generally vary from about 0.1 milligram per kilogram of body weight per day (mg/kg/day) to about mg/kg/day.

In effecting treatment of a patient a pyrazolopyridine derivative can be administered in any form or mode which makes the compound bioavailable in effective amounts, including oral and parenteral routes. For example, the compounds can be administered orally, subcutaneously, intramuscularly, intravenously, transdermally, intranasally, rectally, and the like. Oral administration is generally preferred. One skilled in the art of preparing formulations can readily select the proper form and mode of administration depending upon the particular characteristics of the compound selected, the disease state to be treated, the stage of the disease, and other relevant circumstances.

The compounds can be administered in the form of pharmaceutical compositions or medicaments which are made by combining the pyrazolopyridine derivatives with pharmaceutically acceptable carriers or excipients, the proportion and nature of which are determined by the solubility and chemical properties of the compound selected, the chosen route of administration, and standard pharmaceutical practice. The pyrazolopyridine derivatives while effective themselves, may be formulated and administered in the form of their pharmaceutically acceptable salts for purposes of stability, convenience of crystallization, increased solubility and the like.

The pharmaceutical compositions or medicaments are prepared in a manner well known in the pharmaceutical art. The carrier or excipient may be a solid, semi-solid, or liquid material which can serve as a vehicle or medium for the active ingredient. Suitable carriers or excipients are well known in the art. The pharmaceutical composition may be adapted for oral or parenteral use and may be administered to the patient in the form of tablets, capsules, suppositories, solution, suspensions, or the like.

The pharmaceutical compositions may be administered orally, for example, with an inert diluent or with an edible carrier. They may be enclosed in gelatin capsules or compressed into tablets. For the purpose of oral therapeutic administration, the pyrazolopyridine derivatives may be incorporated with excipients and used in the form of tablets, troches, capsules, elixirs, suspensions, syrups, wafers, chewing gums and the like. These preparations should contain at least 4% of the pyrazolopyridine derivative, the active ingredient, but may be varied depending upon the particular form and may conveniently be between 4% to about 70% of the weight of the unit. The amount of the active ingredient present in compositions is such that a unit dosage form suitable for administration will be obtained.

The tablets, pills, capsules, troches and the like may also contain one or more of the following adjuvants: binders, such as microcrystalline cellulose, gum tragacanth or gelatin; excipients, such as starch or lactose, disintegrating agents such as alginic acid, Primogel, corn starch and the like; lubricants, such as magnesium stearate or Sterotex; glidants, such as colloidal silicon dioxide; and sweetening agents, such as sucrose or saccharin may be added or flavoring agents, such as peppermint, methyl salicylate or orange flavoring. When the dosage unit form is a capsule, it may contain, in addition to materials of the above type, a liquid carrier such as polyethylene glycol or a fatty oil. Other dosage unit forms may contain other various materials which modify the physical form of the dosage unit, for example, as coatings. Thus, tablets or pills may be coated with sugar, shellac, or other enteric coating agents. A syrup may contain, in addition to the active ingredient, sucrose as a sweetening agent and certain preservatives, dyes and colorings and flavors. Materials used in preparing these various compositions should be pharmaceutically pure and non-toxic in the amounts used.

For the purpose of parenteral administration, the pyrazolopyridine derivatives may be incorporated into a solution or suspension. These preparations should contain at least 0.1% of a compound of the invention, but may be varied to be between 0.1 and about 50% of the weight thereof. The amount of the active ingredient present in such compositions is such that a suitable dosage will be obtained.

The solutions or suspensions may also include one or more of the following adjuvants: sterile diluents such as water for injection, saline solution, fixed oils, polyethylene glycols, glycerine, propylene glycol or other synthetic solvents; antibacterial agents such as benzyl alcohol or methyl paraben; antioxidants such

as ascorbic acid or sodium bisulfite; chelating agents such as ethylene diaminetetraacetic acid; buffers such as acetates, citrates or phosphates and agents for the adjustment of toxicity such as sodium chloride or dextrose. The parenteral preparation can be enclosed in ampules, disposable syringes or multiple dose vials made of glass or plastic.

As with any group of structurally related compounds which possess a particular generic utility, certain groups and configurations are preferred in the end-use application. Especially preferred in the use according to the present invention are compounds of formula (1) wherein R and $R_1$ are each independently $(C_1\text{-}C_6)$alkyl, $R_2$ is a phenyl group monosubstituted by nitro, $(C_1\text{-}C_4)$alkyl, chloro or trifluoromethyl, $R_3$ is $(C_1\text{-}C_6)$alkyl, and $R_4$ is hydrogen.

The following compounds are most preferred for the use according to the present invention :

4,7-dihydro-1,6-dimethyl-4-(2-methylphenyl)-3-(2-methylpropyl)-1H-pyrazolo[3,4-b]pyridine-5-carboxylic acid methyl ester,

4,7-dihydro-1,6-dimethyl-4-(2-nitrophenyl)-3-(1-methylethyl)-1H-pyrazolo[3,4-b]pyridine-5-carboxylic acid methyl ester,

4,7-dihydro-1,6-dimethyl-4-(2-nitrophenyl)-3-(1-methylethyl)-1H-pyrazolo[3,4-b]pyridine-5-carboxylic acid ethyl ester,

4,7-dihydro-1,6-dimethyl-4-(2-nitrophenyl)-3-(1-methylethyl)-1H-pyrazolo[3,4-b]pyridine-5-carboxylic acid methyl ester,

4,7-dihydro-1,6-dimethyl-4-(2-nitrophenyl)-3-(2-methylpropyl)-1H-pyrazolo[3,4-b]pyridine-5-carboxylic acid methyl ester,

4,7-dihydro-1,6-dimethyl-4-(3-nitrophenyl)-3-(n-butyl)-1H-pyrazolo[3,4-b]pyridine-5-carboxylic acid methyl ester,

4,7-dihydro-1,6-dimethyl-4-(3-nitrophenyl)-3-(isobutyl)-1H-pyrazolo[3,4-b]pyridine-5-carboxylic acid methyl ester,

4,7-dihydro-1,6-dimethyl-4-(3-nitrophenyl)-3-(cyclobutyl)-1H-pyrazolo[3,4-b]pyridine-5-carboxylic acid methyl ester,

4,7-dihydro-1,6-dimethyl-4-(2-nitrophenyl)-3-(cyclopentyl)-1H-pyrazolo[3,4-b]pyridine-5-carboxylic acid methyl ester,

4,7-dihydro-1,6-dimethyl-4-(3-nitrophenyl)-3-(cyclopentyl)-1H-pyrazolo[3,4-b]pyridine-5-carboxylic acid methyl ester,

4,7-dihydro-1,6-dimethyl-4-(3-nitrophenyl-3- (cyclopentyl)-1H-pyrazolo[3,4-b]pyridine-5-carboxylic acid ethyl ester,

4,7-dihydro-1,6-dimethyl-4-(3-nitrophenyl)-3-(cyclopentyl)-1H-pyrazolo[3,4-b]pyridine-5-carboxylic acid isopropyl ester,

4,7-dihydro-1,6-dimethyl-4-(2-nitrophenyl)-3-(cyclohexyl)-1H-pyrazolo[3,4-b]pyridine-5-carboxylic acid methyl ester,

4,7-dihydro-1,6-dimethyl-4-(3-nitrophenyl)-3-(cyclohexyl)-1H-pyrazolo[3,4-b]pyridine-5-carboxylic acid methyl ester,

4,7-dihydro-1,6-dimethyl-4-(3-nitrophenyl)-3-(cycloheptyl)-1H-pyrazolo[3,4-b]pyridine-5-carboxylic acid methyl ester,

The following example illustrates the use of the pyrazolopyridine derivatives according to the present invention but is not intended to limit the scope thereof in any way.

## EXAMPLE

Antiatherosclerotic and Antihypercholesterolemic Effects of 4,7-Dihydro-1,6-Dimethyl-4-(2-Methylphenvl)-3-(2-Methylpropyl)-1H-Pyrizolo[3,4-b]Pyridine-5-Carboxylic Acid Methyl Ester Monohydrochloride in the Hypercholesterolemic Rabbit

### Methods

Four groups of New Zealand white male rabbits (weighing 1-2 kg) were treated for six weeks according to the following treatment schedule [all injections were in volumes corresponding to 0.1 ml/kg subcutaneously (s.c.)]:

Group 1 maintained on normal rabbit chow; received polyethylene glycol 300 (PEG ) injection twice daily.

Group 2 (Positive Control) maintained on rabbit chow containing 2% by weight cholesterol; received PEG injection twice daily.

Group 3 maintained on rabbit chow containing 2% by weight cholesterol; received 4,7-dihydro-1,6-dimethyl-4-(2-methylphenyl)-3-(2-methylpropyl)-1H-pyrozolo[3,4-b]pyridine-5-carboxylic acid methyl ester monohydrochloride (compound 1A) at a dose of 1 mg/kg in PEG twice daily.

Group 4 maintained on rabbit chow containing 2% by weight cholesterol; received compound 1A at a dose of 10 mg/kg in PEG twice daily.

The body weight of each rabbit was measured weekly. At three week and six week intervals after the start of treatment, blood samples were obtained from each rabbit and determinations of platelet aggregation, serum cholesterol, triglycerides, electrolyte concentrations and hematolcgical parameters were performed.

Six weeks after the start of treatment, the animals were sacrificed. Aortic arches and thoracic aortas were removed and were dissected free of extraneous tissues. The aortic arches were weighed, homogenized in isopropanol, and determinations of cholesterol, triglycerides and electrolyte concentrations were performed. Thoracic aortas were slit down the ventral surface and stained for lipoproteins and lipids by immersion for 5 min in Sudan Black according to the method of Gradwohl (Gradwohl's Clinical Laboratory Methods and Diagnosis (1980) Vol. 1, Edition 8, Chapter 25, pg 771; Sonnenwerth and Jarett, editors, The C.V. Mosby Company, St. Louis, Toronto, London). The percentage of luminal surface covered by atherosclerotic plaque was assessed by "cut-weight" planimetry of the projected image according to the method of Willis et al. (Arteriosclerosis 5, 250 (1985)) whereby the photographic image was projected at a magnification of 7 to 10 and the outer border of the tissue and the boundary between plaque and uninvolved tissue were traced and the appropriate areas were cut out and weighed.

## Results

No significant differences between the mean body weights of the different treatment groups was apparent.

As shown in Table 1, the hypercholesterolemic diet causes a dramatic increase in serum cholesterol, aortic arch cholesterol, and percent of thoracic aorta involved in atherosclerotic plaque formation in rabbits. However, the levels of serum cholesterol, aortic arch cholesterol and percent of thoracic aorta covered by plaque are all significantly (p<.01) reduced in the groups treated with Compound 1A at both the 1 mg/kg dose (Group 3) and the 10 mg/kg dose (Group 4) as compared to the corresponding values in the non-drug treated positive control group (Group 2).

TABLE 1

Antihypercholesterolemic and Antiatherosclerotic Effects of Treatment of Hypercholesterolemic Rabbits With Compound 1A

| Treatment Group, 6 Weeks Treatment | 1 | 2 | 3 | 4 |
|---|---|---|---|---|
| Hypercholesterolemic Diet | | X | X | X |
| Dose Compound 1A, mg/kg bid | 0 | 0 | 1 | 10 |
| Serum Cholesterol, mg/dl (after 6 wks treatment) | 65±13[a]* | 2475±434 | 1919±345* | 1774±300* |
| Aortic arch cholesterol, µg/mg wet wt. | 3.36±0.48* | 15.82±4.64 | 9.57±1.38* | 9.27±1.91* |
| % of thoracic arota covered by plaque | 1.2±1.79* | 70.57±9.54 | 34.33±19.03* | 36.71±9.95* |

[a]mean ± standard deviation
*p <.01 as compared to Group 2

These results clearly demonstrate the antihypercholesterolemic and antiatherosclerotic effects of Compound 1A.

**Claims**

1. The use in the manufacture of a medicament, for treating a patient for atherosclerosis, of a pyrazolopyridine derivative of the formula

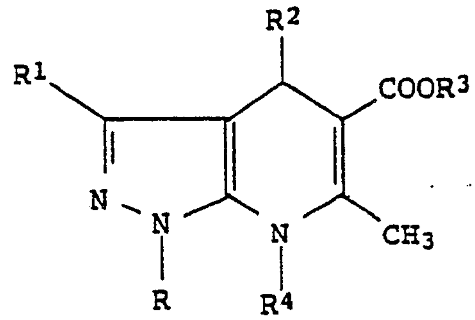

wherein

R is hydrogen, $(C_1\text{-}C_6)$alkyl, phenyl, or phenyl which is optionally substituted by from 1, 2 or 3 substituents selected from $(C_1\text{-}C_4)$alkyl, halo$(C_1\text{-}C_4)$alkyl, $(C_1\text{-}C_4)$alkoxy, chloro, fluoro, bromo, nitro, and $(C_1\text{-}C_6)$alkoxy-carbonyl, or phenyl$(C_1\text{-}C_4)$alkyl, wherein the phenyl group is optionally substituted as above;

$R^1$ is hydrogen, $(C_1\text{-}C_6)$alkyl, $(C_3\text{-}C_7)$cycloalkyl, phenyl, or phenyl which is optionally substituted by from 1, 2 or 3 substituents selected from $(C_1\text{-}C_4)$alkyl, halo$(C_1\text{-}C_4)$alkyl, $(C_1\text{-}C_4)$alkoxy, chloro, fluoro, bromo, nitro, and $(C_1\text{-}C_6)$alkoxy-carbonyl, or phenyl$(C_1\text{-}C_4)$alkyl, wherein the phenyl group is optionally substituted as above;

$R^2$ is a phenyl group substituted by one or two substituents selected from nitro, $(C_1\text{-}C_4)$alkoxy, $(C_1\text{-}C_4)$-alkyl, chloro or trifluoromethyl;

$R^3$ is $(C_1\text{-}C_6)$alkyl or $(C_1\text{-}C_4)$alkoxy$(C_1\text{-}C_6)$alkyl;

$R^4$ is hydrogen, methyl or benzyl; or the physiologically acceptable salts thereof.

2. The use in the manufacture of a medicament, for treating a patient for hypercholesterolemia, of a pyrazolopyridine derivative of the formula

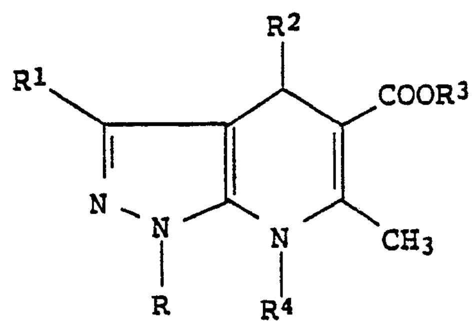

wherein

R is hydrogen, $(C_1\text{-}C_6)$alkyl, phenyl, or phenyl which is optionally substituted by from 1, 2 or 3 substituents selected from $(C_1\text{-}C_4)$alkyl, halo$(C_1\text{-}C_4)$alkyl, $(C_1\text{-}C_4)$alkoxy, chloro, fluoro, bromo, nitro, and $(C_1\text{-}C_6)$alkoxy-carbonyl, or phenyl$(C_1\text{-}C_4)$alkyl, wherein the phenyl group is optionally substituted as above;

$R^1$ is hydrogen, $(C_1\text{-}C_6)$alkyl, $(C_3\text{-}C_7)$cycloalkyl, phenyl, or phenyl which is optionally substituted by from 1, 2 or 3 substituents selected from $(C_1\text{-}C_4)$alkyl, halo$(C_1\text{-}C_4)$alkyl, $(C_1\text{-}C_4)$alkoxy, chloro, fluoro, bromo, nitro, and $(C_1\text{-}C_6)$alkoxy-carbonyl, or phenyl$(C_1\text{-}C_4)$alkyl, wherein the phenyl group is optionally substituted as above;

$R^2$ is a phenyl group substituted by one or two substituents selected from nitro, $(C_1\text{-}C_4)$alkoxy, $(C_1\text{-}C_4)$-alkyl, chloro or trifluoromethyl;

$R^3$ is $(C_1\text{-}C_6)$alkyl or $(C_1\text{-}C_4)$alkoxy$(C_1\text{-}C_6)$alkyl;

$R^4$ is hydrogen, methyl or benzyl; or the physiologically acceptable salts thereof.

3. The use in the manufacture of a medicament according to Claim 2 for additionally treating a patient for atherosclerosis.

4. The use in the manufacture of a medicament according to Claim 1, 2 or 3 wherein the

pyrazolopyridine derivative is selected from the group consisting of :
4,7-dihydro-1,6-dimethyl-4-(2-methylphenyl)-3-(2-methylpropyl)-1H-pyrazolo[3,4-b]pyridine-5-carboxylic acid methyl ester;
4,7-dihydro-1,6-dimethyl-4-(2-nitrophenyl)-3-(1-methylethyl)-1H-pyrazolo[3,4-b]-pyridine-5-carboxylic acid methyl ester;
4,7-dihydro-1,6-dimethyl-4-(2-nitrophenyl)-3-(1-methylethyl)-1H-pyrazolo[3,4-b]pyridine-5-carboxylic acid ethyl ester;
4,7-dihydro-1,6-dimethyl-4-(2-nitrophenyl)-3-(1-methylethyl)-1H-pyrazolo[3,4-b]pyridine-5-carboxylic acid methyl ester;
4,7-dihydro-1,6-dimethyl-4-(2-nitrophenyl)-3-( -methylpropyl)-1H-pyrazolo[3,4-b]pyridine-5-carboxylic acid methyl ester;
4,7-dihydro-1,6-dimethyl-4-(3-nitrophenyl)-3-(n-butyl)-1H-pyrazolo[3,4-b]pyridine-5-carboxylic acid methyl ester;
4,7-dihydro-1,6-dimethyl-4-(3-nitrophenyl)-3-(isobutyl)-1H-pyrazolo[3,4-b]pyridine-5-carboxylic acid methyl ester
4,7-dihydro-1,6-dimethyl-4-(3-nitrophenyl)-3-(cyclobutyl)-1H-pyrazolo[3,4-b]pyridine-5-carboxylic acid methyl ester;
4,7-dihydro-1,6-dimethyl-4-(2-nitrophenyl)-3-(cyclopentyl)-1H-pyrazolo[3,4-b]pyridine-5-carboxylic acid methyl ester;
4,7-dihydro-1,6-dimethyl-4-(3-nitrophenyl)-3-(cyclopentyl)-1H-pyrazolo[3,4-b]pyridine-5-carboxylic acid methyl ester;
4,7-dihydro-1,6-dimethyl-4-(3-nitrophenyl)-3-(cyclopentyl)-1H-pyrazolo[3,4-b]pyridine-5-carboxylic acid ethyl ester;
4,7-dihydro-1,6-dimethyl-4-(3-nitrophenyl)-3-(cyclopentyl)-1H-pyrazolo[3,4-b]pyridine-5-carboxylic acid isopropyl ester;
4,7-dihydro-1,6-dimethyl-4-(2-nitrophenyl)-3-(cyclohexyl)-1H-pyrazolo[3,4-b]pyridine-5-carboxylic acid methyl ester;
4,7-dihydro-1,6-dimethyl-4-(3-nitrophenyl)-3-(cyclohexyl)-1H-pyrazolo[3,4-b]pyridine-5-carboxylic acid methyl ester;
4,7-dihydro-1,6-dimethyl-4-(3-nitrophenyl)-3-(cycloheptyl)-1H-pyrazolo[3,4-b]pyridine-5-carboxylic acid methyl ester.

European Patent Office

# EUROPEAN SEARCH REPORT

Application Number

EP 88 40 2163

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | BIOCHEMICAL PHARMACOLOGY, vol. 28, 1979, pages 1217-1219, Pergamon Press Ltd, GB; R.J. MORIN et al.: "Effects of 4-aminopyrazolopyrimidine on rabbit plasma cholesterol, platelet 3-hydroxy-3-methyl-glutaryl-coenzyme a reductase and platelet aggregation" * Page 1219 * | 1-4 | A 61 K 31/44 |
| Y,D | EP-A-0 107 619 (SHIONOGI AND CO., LTD) * Page 1, paragraph 1; page 13, line 23 - page 14, line 2; claim 1 * | 1-4 | |
| Y | US-A-4 565 823 (I. OHATA) * Column 1, line 1 - column 2, line 10 * | 1-4 | |
| Y | JOURNAL OF CARDIOVASCULAR PHARMACOLOGY, vol. 10, no. 1, 1987, pages 30-37, Raven Press, New York, US; J.C. WYNSEN et al.: "Cardiovascular actions of a new dihydropyridine calcium antagonist, 8363-S: comparison with nifedipine and nicardipine in awake, unsedated dogs" * Whole article * | 1-4 | TECHNICAL FIELDS SEARCHED (Int. Cl.4)<br>A 61 K 31/00 |
| A | FR-A-2 182 914 (KYORIN SEIYAKU K.K.) * Page 1, lines 1-16; claim 1 * | 1-4 | |

The present search report has been drawn up for all claims

| Place of search | Date of completion of the search | Examiner |
|---|---|---|
| THE HAGUE | 06-03-1989 | GERLI P.F.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)